# EUROPEAN PATENT SPECIFICATION

(11) **EP 0 994 844 B1**
(45) Date of publication and mention of the grant of the patent: **31.07.2002**
(21) Application number: 97945201.8
(22) Date of filing: 09.09.1997
(51) Int. Cl.: C07C 69/653, C08F 220/22, G02B 1/04

(54) **HIGH INDEX OF REFRACTION MONOMERS**
MONOMERE MIT HOHEM BRECHUNGSINDEX
MONOMERES A INDICE DE REFRACTION ELEVE

(30) Priority: 09.05.1997 US 853981
(43) Date of publication of application: 26.04.2000
(73) Proprietor: MINNESOTA MINING AND MANUFACTURING COMPANY, St. Paul, Minnesota 55133-3427 (US)
(72) Inventor: OLSON, David, B., Saint Paul, MN 55133-3427 (US)
(74) Representative: VOSSIUS & PARTNER
(86) International application number: US9715861
(87) International publication number: WO9850340

(56) References cited:
- DE-A- 2 161 526
- PATENT ABSTRACTS OF JAPAN vol. 11, no. 159 (C-423), 22 May 1987 & JP 61 286347 A (MITSUI TOATSU CHEM INC), 16 December 1986,

## Description

The invention relates to alkyl-substituted, brominated aromatic ester (meth)acrylate monomers.

### BACKGROUND

High index of refraction monomers are useful in the production of optical materials and optical products. High index of refraction monomers can be cured or polymerized, alone or in combination with other reactive materials, to produce optical products such as films, lenses, optical fibers, microreplicated materials, and other articles useful to control the flow and intensity of light. United States Patent No. 4,578,445, for example, describes the use of high index of refraction halogen-containing materials for the production of optical lenses, see also, United States Patent Nos. 4,721,377, 4,812,032, and 5,424,339. In order to continually improve such optical products, or the processes for preparing them, there is an ongoing need to develop new and improved high index of refraction monomers.

Some halogenated aromatic (meth)acrylate monomers have been found to be useful as high index of refraction monomers. These monomers exhibit desirable optical qualities, but are generally relatively high melting materials that exist as solids at temperatures near room temperature (e.g., in the range from about 20 to 25°C), and often have melting points significantly above room temperature. Because these monomers exist as solids at room temperature, polymerizable compositions containing these monomers can require heating to a temperature above room temperature to be processed and formed into a high index of refraction optical product. The need to heat monomers above room temperature during processing adds significant complication and expense to the processing of the monomer into an optical product. Processing lines must be heated, which can require large amounts of energy, cause the evolution of high volatile organic compounds, and sometimes rupture tubing connections. Also, if complete and uniform heating is not consistently maintained, the monomer can crystallize within the curable composition during processing, resulting in the production of nonuniform and unusable product, and thereby resulting in high amounts of waste product. Furthermore, maintaining the polymerizable composition at an appropriate processing temperature can potentially cause prepolymerization of the monomeric composition. Of course, all of these added process requirements and negative consequences increase the cost of producing high index of refraction products from these high melting monomers.

In light of these processing requirements, it would be especially desirable to identify polymerizable monomers that have physical properties including a relatively high index of refraction, and additionally, a relatively low melting point, e.g., a high index of refraction monomer that can be processed at temperatures at or near normal room temperature (between about 20°C and 25°C).

### SUMMARY OF THE INVENTION

The present invention provides polymerizable, brominated monomers that have a relatively high index of refraction; i.e., at least 1.55. Preferred monomers of the present invention can also have relatively low melting temperatures; i.e., below 60°C, more preferably below 35°C or 30°C, and most preferably, the monomer exists as a liquid at relatively low temperatures such as at normal room temperature. Because these monomers have relatively low melting temperatures, they can, by themselves or in combination with other polymerizable materials, be processed at relatively low temperatures (e.g., at or near room temperature), thereby reducing the expense and complication associated with heating the monomers during processing. By "processing" it is meant that the monomers can be blended, pumped, and otherwise handled prior to polymerization and manufacturing to produce a polymer or optical product. Further, even those monomers of the present invention that have melting points above room temperature (e.g., from 30°C to 60°C) can be desirable, because these monomers can often be easily dissolved in other liquids without significantly raising the melting point of the liquid.

An aspect of the present invention relates to an alkyl-substituted brominated aromatic ester (meth)acrylate monomer having an index of refraction of at least 1.55, preferably at least 1.56. Examples of such monomers include those having the general formula: 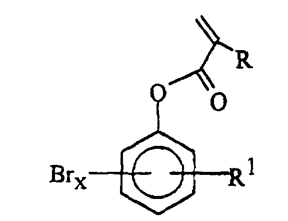 wherein x is from 1 to 4, R is -H or -CH₃, and R¹ is a straight or branched alkyl having at least two carbon atoms.

As used within the present description, "monomer" refers to a monomer on an individual scale, and can also refer collectively to a composition of such monomers on a macroscopic scale such that the composition can be described as having a physical state of matter (e.g., liquid, solid, etc.) and physical properties (e.g., melting point, viscosity, glass transition temperature (of a polymeric form), and index of refraction).

"Melting point," as used with respect to the monomer, refers to the temperature at which the monomer passes from a solid state to a liquid state, as measured at atmospheric pressure. Melting point can be measured by methods known in the chemical art, for example, using a Thomas-Hoover Melting Point Apparatus, available from Thomas Scientific, Swedesboro, NJ.

"Index of refraction," or "refractive index," refers to the absolute refractive index of a material (e.g., a monomer), which is understood to be the ratio of the speed of electromagnetic radiation in free space to the speed of the radiation in that material, with the radiation being of sodium yellow light at a wavelength of about 583.9 nanometer (nm). Index of refraction can be measured by known methods, and is generally measured using an Abbe Refractometer.

"(Meth)acrylate" refers to both acrylate and methacrylate compounds.

### DETAILED DESCRIPTION

Monomers of the present invention comprise alkyl-substituted brominated phenolic ester (meth)acrylate monomers (also referred to herein as "the monomer" or "the brominated monomer"). The monomers exhibit desirable physical properties due to their chemical structure, which includes an aromatic ester (meth)acrylate, the presence of bromine, and the presence of an alkyl group.

The substituents of the aromatic portion of the monomer can affect or substantially determine the physical properties of the monomer, including index of refraction and melting point. These properties are believed to be affected by the chemical composition of the substituents, the size and number of such substituents, and the location of such substituents on the monomeric structure. While wishing not to be bound by theory, the alkyl substituent (e.g., its size, position, and chemical composition) is believed to affect the melting point of the monomer. Further, the position of the alkyl group on the aromatic portion of the monomer can affect the size of the alkyl group required to provide a desired physical property such as melting point. That is, preferred alkyl groups in the para position that provide a desired melting point or index of refraction may be of a size range different from preferred alkyl groups in the meta or ortho positions that also provide the desired property.

The chemical composition and position of the alkyl on the aromatic ring can affect the index of refraction of the monomer. Thus, while wishing not to be bound by theory, and while observing that monomers having relatively larger alkyl groups can be useful within the present invention, it can be said that in a very general sense larger alkyl groups can result in a lower index of refraction.

The index of refraction of the monomer can also be affected by the presence and position of bromine on the monomer. It is generally understood that bromine increases the index of refraction of the monomer. Bromine can be present on the monomer at any useful position and in any amount sufficient to provide a monomer having an index of refraction of at least 1.55. This can be accomplished, for example, by having at least two bromines directly attached to the aromatic portion of the monomer. Often, the position of the bromine can be a function of the materials and process used to prepare the brominated monomer (described *infra*). Also, the position of the bromine can depend at least in part on the position of the alkyl. If the alkyl is attached at the 4 position relative to the ester substituent, bromines are preferably at the 2 and 6 positions, and, if the alkyl is at the 2 position, bromines are preferably at the 4 and 6 positions.

Examples of useful brominated monomers include those having the structure of general formula 1: 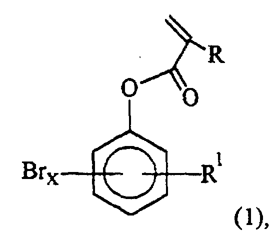 wherein:
R can be hydrogen (-H) or methyl (-CH₃);
x can be in the range from 1 to 4, and is preferably 2; and
R¹ can be a straight or branched alkyl having two or more carbon atoms, and being of a size and chemical composition that, in combination with other substituents of the monomer, will provide a monomer having an index of refraction of at least 1.55. R¹ can be positioned either ortho, meta, or para to the ester.

Some preferred brominated monomers are of such a chemical composition, i.e., contain bromine and an appropriate alkyl group, that the monomers can be processed at a relatively low temperature. Specifically, these preferred monomers can have a melting point below 60°C, more preferably below 35°C or 30°C, even more preferably below 25°C, and most preferably the monomer remains in a liquid state at about room temperature (23°C).

A property that is related to melting point of the monomer is viscosity. The monomers of the present invention preferably are of a viscosity that allows the monomer or a polymerizable composition thereof to be processed at room temperature. Although viscosities outside of the following ranges can be useful, preferred viscosities of the monomer can be in the range from about 100 to 5000 centipoise (cps), more preferably in the range from about 300 to 4000 cps, as measured at 23°C. Viscosity of a liquid monomer can be measured by methods that are known in the chemical art, for example using a viscometer such as a Brookfield viscometer.

Examples of monomers having the above-identified index of refraction and preferred melting point include those substituted with an alkyl group located ortho to the ester substituent: 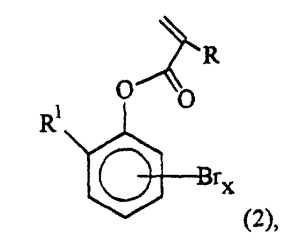 wherein R, x, and R¹ are as defined *supra*. In a particularly preferred embodiment of this monomer, bromines are located at the 4 and 6 positions on the aromatic ring, ortho and para to the ester substituent:

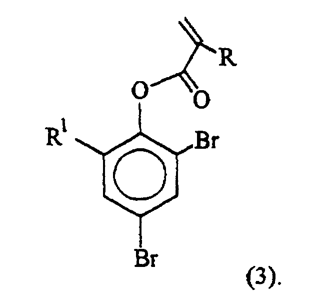

Particular monomers according to formula 3 include 4,6-dibromo-2-alkyl phenolic ester (meth)acrylates wherein the alkyl has from 3 to 4 carbons, including the following:
4,6-dibromo-2-sec-butyl phenyl (meth)acrylate: 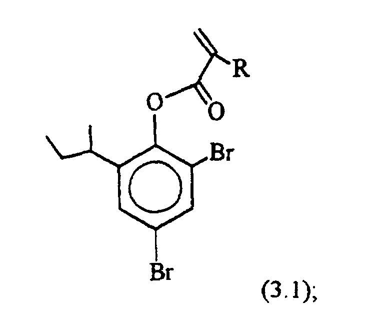 4,6-dibromo-2-tert-butyl phenyl (meth)acrylate: 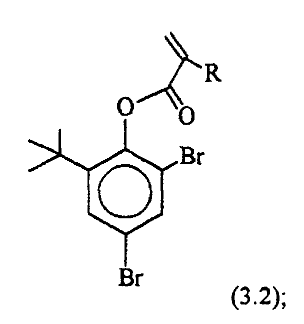 and
4,6-dibromo-2-isopropyl phenyl (meth)acrylate:

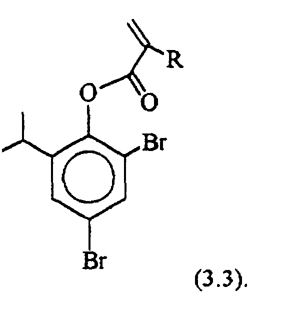

Monomers of the present invention can be prepared by any method generally useful in preparing brominated phenolic compounds, and in particular, alkyl-substituted brominated phenolic ester (meth)acrylates. Such methods are well known in the chemical art. By one particular method, an alkyl-substituted phenol can be brominated to produce a brominated alkylphenol. Alkylphenols are commercially available from Schenectady International Inc., Chemical Division, Schenectady, NY. Such alkyl-substituted phenols can be brominated by methods that are generally known in the chemical art, and as described, for example, in the Kirk-Othmer Encyclopedia of Chemical Technology, Volume 4, 543 (4^{th} ed. 1992). Such a process as exemplified with ortho-substituted alkyl phenols is shown as follows: 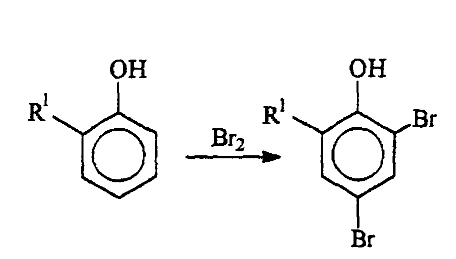 The brominated alkylphenol can be esterified to produce an alkyl-substituted brominated phenolic ester (meth)acrylate by reaction with an appropriate acid chloride. The reaction between an alcohol and an acid chloride is well known in the chemical art and is described, for example, in the Kirk-Othmer Encyclopedia of Chemical Technology, Volume 9, 769 (4^{th} ed. 1992); see also United States Patent No. 3,845,102. Inhibitors, such as phenothiazine or 4-methoxyphenol (MEHQ), can be used in such an amount to provide protection from pre-polymerization of the monomer during its synthesis and storage, while not excessively influencing the subsequent polymerization. With respect to the monomer of the present invention, the brominated alkylphenol can be reacted with (meth)acryloyl chloride as shown:

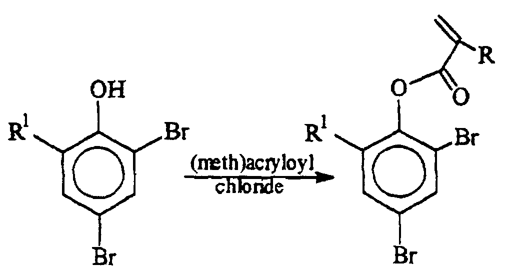

The brominated monomer of the invention, alone or in combination with other materials such as unsaturated polymerizable comonomers, can be included in a polymerizable composition that can be polymerized or copolymerized to produce useful polymers or polymeric or copolymeric materials. As used within the present description the term "polymerizable" refers to a composition or monomer capable of polymerizing or copolymerizing (e.g., via unsaturated moieties) to produce a higher molecular weight material such as a polymer or polymeric material. "Polymer" or "polymeric material" refers to a material prepared from the reaction of one or more unsaturated monomers, oligomers, or polymers, etc., and includes, e.g., dimers, trimers, oligomers, pre-polymers, copolymers, homopolymers, etc. The production of polymers and optical products from high index of refraction, polymerizable materials, is described, for example, in United States Patent Nos. 5,175,030, 5,183,597, and 5,591,527.

The invention will be more fully appreciated with reference to the following non-limiting examples in which the reaction components are given as grams used or as weight percents (wt %), based on the total weight of the reaction mixtures which are nominally 100 weight %. Dimensions in English units are nominal and conversion to metric units is approximate.

### Examples

### Preparation of 4,6-dibromo-2-sec-butyl phenol (DBsBP)

In an appropriately sized round bottom flask equipped with a mechanical stirrer, condenser, nitrogen cap, addition funnel and temperature probe, 850g (grams) of 2-sec-butylphenol was mixed with 5097g of deionized water. The mixture was stirred with a mechanical mixer and purged with nitrogen for about 10 minutes. 1881g bromine was added to the mixture drop-wise through the addition funnel. The reaction temperature was maintained at about 30°C or less using an ice bath. Following the addition of the bromine, the reaction mixture was stirred for 30 minutes at room temperature. Reaction completion was determined by gas chromatography, by monitoring the disappearance of the starting material and of monobrominated species.

Upon completion of the reaction, 4487g of ethyl acetate was added. The mixture was stirred for 15 minutes and then allowed to phase split. The bottom (aqueous) layer was removed and 750.5g of a 13 wt% aqueous sodium hydrosulfite solution was added. The mixture was stirred well and then allowed to phase split. The bottom (aqueous) layer was removed and 856.4g of a 13 wt% aqueous sodium chloride solution was added. The mixture was stirred well and then allowed to phase split. The bottom (aqueous) layer was removed and solvent was stripped from the top layer using a rotary evaporator.

The crude product was then distilled using a distillation head and vigreux column. The product distilled at 0.1 mm Hg, a pot temperature of 151°C, and a head temperature of 97°C. This procedure provided approximately 1500g of DBsBP.

### Preparation of 4,6-dibromo-2-isopropyl phenol (DBiPP)

The procedure described for the preparation of DBsBP was followed using 800g of 2-isopropylphenol instead of the 2-sec-butylphenol, 5291g of water, 1953g of bromine, 4658g of ethyl acetate, 780g of 13% (w/w) aqueous sodium hydrosulfite and 890g of 13% (w/w) aqueous sodium chloride to produce 1598 g of DBiPP.

### Preparation of 4,6-dibromo-2-t-butyl phenol (DBtBP)

The procedure described for the preparation of DBsBP was followed using the following materials; 330 grams of 2-t-butylphenol instead of the 2-sec-butylphenol, 5500 grams deionized water, 730 grams bromine, 329 grams ethyl acetate, 3620 grams of a 9% (w/w) aqueous sodium hydrosulfite solution, and 3300 grams of a saturated aqueous sodium chloride solution, to produce 573 grams DBtBP.

### Example 1 Synthesis of 4,6-dibromo-2-sec-butyl phenyl acrylate (DBsBPA)

In an appropriately sized round bottom flask equipped with a mechanical stirrer, condenser, addition funnel and temperature probe, 140g of 4,6-dibromo-2-sec-butyl phenol, 360g of t-butyl methyl ether, 55.2g triethylamine, and 0.02g phenothiazine were mixed (in these examples, the base used was triethylamine; however, a stoichiometric amount of any other appropriate base could also be used, such as sodium hydroxide or pyridine, among others). 47.3g of acryloyl chloride was added dropwise and, using an ice bath, the reaction temperature was maintain below 20°C. The reaction was run to completion, taking approximately 30 minutes.

The product was then worked up by sequential washings with deionized water (257g); 0.7% (w/w) aqueous hydrochloric acid (51g); 16.1% (w/w) aqueous sodium carbonate (59.6g); and 8.3%(w/w) aqueous sodium chloride (54.5g). Solvent was removed using a rotary evaporator and the crude product was vacuum distilled to yield 155grams (94%) of DBsBPA.

### Example 2 Synthesis of 4,6-dibromo-2-sec-butyl phenyl methacrylate (DBsBPMA)

A procedure similar to that described in Example 1 was used to prepare 4,6-dibromo-2-sec-butyl phenyl methacrylate, except methacryloyl chloride was used in place of acryloyl chloride.

### Example 3 Synthesis of 4,6-dibromo-2-t-butyl phenyl acrylate (DBtBPA)

A procedure similar to that described in Example 1 was used to prepare 4,6-dibromo-2-t-butyl phenyl acrylate, except 4,6-dibromo-2-t-butyl phenol (DBtBP) was used instead of 4,6-dibromo-2-sec-butyl phenol.

### Example 4 Synthesis of 4,6-dibromo-2-isopropyl phenyl acrylate (DBiPPA)

A procedure similar to that described in Example 1 was used to prepare 4,6-dibromo-2-isopropyl phenyl acrylate, except 4,6-dibromo-2-isopropyl phenol (DBiPP) was used in place of 4,6-dibromo-2-sec-butyl phenol. This sample would solidify over a period of days or weeks. Therefore, viscosity data from this example were taken prior to solidification, and melting point data were taken following solidification.

### Example 5 Synthesis of 4,6-dibromo-2-isopropyl phenyl methacrylate (DBiPPMA)

A procedure similar to that described in Example 1 was used to prepare 4,6-dibromo-2-isopropyl phenyl acrylate, except 4,6-dibromo-2-isopropyl phenol (DBiPP) was used in place of4,6-dibromo-2-sec-butyl phenol, and methacryloyl chloride was used in place of acryloyl chloride. This sample started out as a liquid at room temperature and thereafter solidified over a period of days or weeks.

### Comparative Example 1 - 2,6-dibromo-4-nonyl phenyl acrylate (DBNPA)

In an appropriately sized round bottom flask equipped with a mechanical stirrer, condenser, nitrogen cap, addition funnel and temperature probe, 44g of 4-nonylphenol and 180g of deionized water were mixed. To this stirred mixture, 77.4g of bromine was added dropwise being careful to keep the reaction temperature below 30°C. After the addition of the bromine, the mixture was allowed to react for about an hour. Once the reaction was complete, as determined by gas chromatography, the product was taken up into an organic phase of 160g ethyl acetate. The organic phase was then washed with sequential washings of 13% (w/w) aqueous sodium hydrosulfite (26.5g) and 13% (w/w) aqueous sodium chloride (30.2g). The ethyl acetate was then stripped on a rotary evaporator and the crude product vacuum distilled using a short vigreux column to yield approximately 66g 2,6-dibromo-4-nonylphenol (DBNP).

In an appropriately sized round bottom flask equipped with a mechanical stirrer, condenser, addition funnel, and temperature probe, 30.5g of 2,6-dibromo-4-nonylphenol, 64g of t-butyl methyl ether, 9.8g of triethylamine, and 0.005g of phenothiazine were mixed. To this stirred mixture, 8.4g of acryloyl chloride was added over a period of 30 minutes being careful to keep the reaction temperature below 35°C. After the addition of the acryloyl chloride, the mixture was allowed to react at room temperature (approximately 25°C) for a period of 2 hours at which point gas chromatography analysis indicated a complete conversion of the 2,6-dibromo-4-nonylphenol to 2,6-dibromo-4-nonyl phenyl acrylate (DBNPA). The product was then worked up with sequential washings of deionized water (45.6g); 0.7% (w/w) aqueous hydrochloric acid (8.9g); 16.4% (w/w) aqueous sodium carbonate (10.4g) and 8.7% (w/w) aqueous sodium chloride (9.5g). The organic layer was then dried over magnesium sulfate and the solvent stripped in vacuum to yield approximately 32g of 2,6-dibromo-4-nonyl phenyl acrylate.

### Comparative Example 2 - 4,6-dibromo-2-dodecyl phenyl acrylate (oDBDPA)

In an appropriately sized round bottom flask equipped with a mechanical stirrer, condenser, nitrogen cap, addition funnel and temperature probe, 19.6g of melted 2-n-dodecyl phenol and 67.3g of deionized water were mixed and heated to 45°C. The reaction flask was purged with a stream of nitrogen. To the heated mixture, 24.8g of bromine was added over 30 minutes and the mixture was allowed to react for 2 hours at 45°C. Two 6g additions of bromine were added to the heated reaction mixture at one hour intervals in order to complete the reaction. Once the reaction was complete, as determined by gas chromatography, the product was taken up into an organic phase of 59.8g ethyl acetate. The organic phase was then washed with sequential washings of 13% (w/w) aqueous sodium hydrosulfite (9.9g) and 13% (w/w) aqueous sodium chloride (11.3g). The organic layer was dried over magnesium sulfate and then the solvent stripped under vacuum to yield approximately 30.3g of a white solid 4,6-dibromo-2-dodecyl phenol (oDBDP).

In an appropriately sized round bottom flask equipped with a mechanical stirrer, condenser, addition funnel and temperature probe, 25g of 4,6-dibromo-2-dodecylphenol, 47.2g of t-butyl methyl ether,7.2g of triethylamine, and 0.004g of phenothiazine were mixed. To this stirred mixture, 6.2g of acryloyl chloride was added over a period of 30 minutes being careful to keep the reaction temperature below 35°C. After the addition of the acryloyl chloride, the mixture was allowed to react at room temperature (approximately 25°C) for a period of 2 hours at which point gas chromatography analysis indicated a complete conversion of the 4,6-dibromo-2-dodecylphenol to 4,6-dibromo-2-dodecyl phenyl acrylate (oDBDPA). The product was then worked up with sequential washings of deionized water (33.7g); 0.7% (w/w) aqueous hydrochloric acid (6.5g); 16.4% (w/w) aqueous sodium carbonate (7.7g) and 8.7% (w/w) aqueous sodium chloride (7.0g). The organic layer was then dried over magnesium sulfate and the solvent stripped in vacuum to yield approximately 23.5 g of 4,6-dibromo-2-dodecyl phenyl acrylate.

### Comparative Example 3 - 2,6-dibromo-4-dodecyl phenyl acrylate (pDBDPA)

In an appropriately sized round bottom flask equipped with a mechanical stirrer, condenser, nitrogen cap, addition funnel and temperature probe, 52.0g of 4-n-dodecyl phenol and 178.5 g of deionized water were mixed and heated to 40°C. The reaction flask was purged with a stream of nitrogen. To the heated mixture, 65.9 g of bromine was added over 30 minutes and the mixture was allowed to react at 40°C until completion. Once the reaction was complete, as determined by thin layer chromatography, the product was taken up into an organic phase of 158.98 ethyl acetate. The organic phase was then washed with sequential washings of 13% (w/w) aqueous sodium hydrosulfite (26.3g) and 13% (w/w) aqueous sodium chloride (30.0g). The organic layer was dried over magnesium sulfate arid then the solvent stripped under vacuum to yield approximately 82g of a light amber oily 2,6-dibromo-4-dodecyl phenol (pDBDP).

In an appropriately sized round bottom flask equipped with a mechanical stirrer, condenser, addition funnel and temperature probe, 78 grams of4,6-dibromo-2-dodecylphenol, 196.3g of t-butyl methyl ether, 22.5g of triethylamine, and 0.011g of phenothiazine were mixed. To this stirred mixture, 19.3g of acryloyl chloride was added over a period of 30 minutes being careful to keep the reaction temperature at approximately 30°C. After addition of the acryloyl chloride, the mixture was allowed to react at room temperature (approximately 25°C) for a period of 1 hour at which point thin layer chromatography analysis indicated a complete conversion of the 2,6-dibromo-4-dodecylphenol to 2,6-dibromo-4-dodecyl phenyl acrylate (pDBDPA). The product was then worked up with sequential washings of deionized water (105g); 0.7% (w/w) aqueous hydrochloric acid (20.48); 16.4% (w/w) aqueous sodium carbonate (24g) and 8.7% (w/w) aqueous sodium chloride (21.9g). The organic layer was then dried over magnesium sulfate and the solvent stripped in vacuum to yield approximately 83g of 2,6-dibromo-4-dodecyl phenyl acrylate.

**Table I**

| Physical Properties | | | | |
|---|---|---|---|---|
| **Example** | **Name** | **Index of Refraction** | **Viscosity (cps) @ 23°C** | **Melting Point (°C)** |
| 1 | DBsBPA | 1.5620 | 420 | R.T. liquid |
| 2 | DBsBPMA | 1.5567 | 320 | R.T. liquid |
| 3 | DBtBPA | 1.5685 | 3700 | R.T. liquid |
| 4 | DBiPPA | 1.5665 | 360 | 52-53°C |
| 5 | DBiPPMA | 1.5580 | not taken | not taken |
| Comp. 1 | DBNPA | 1.5436 | 3800 | R.T. liquid |
| Comp. 2 | oDBDPA | 1.5263 | not taken | 36-40°C |
| Comp. 3 | pDBDPA | 1.5330 | 3840 | R.T. liquid |

### Measurement of Refractive Index

The refractive index of monomers was measured using an Abbe Refractometer, made by Erma Inc.,of Tokyo Japan, and distributed by Fisher Scientific.

### Measurement of Viscosity

Viscosities were measured using a Brookfield Model LV viscometer set at 30 RPM and using a #3 spindle.

## Claims

1. A monomer of the general formula: 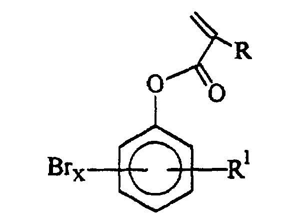 wherein x is from 1 to 4, R is -H or -CH₃, and R¹ is a straight or branched alkyl having at least two carbon atoms, and wherein the monomer has an index of refraction of at least 1.55.

2. The monomer of claim 1, wherein the index of refraction is at least 1.555.

3. The monomer of claim 1, wherein the index of refraction is at least 1.56.

4. The monomer of claim 1, wherein the melting point of the monomer is below 60°C.

5. The monomer of claim 1, wherein the melting point of the monomer is below 30°C.

6. The monomer of claim 1, wherein the melting point of the monomer is in the range from 20 to 25°C.

7. The monomer of claim 1, wherein R¹ is a straight or branched alkyl having from 3 to 4 carbon atoms.

8. The monomer of claim 7, wherein x is 2.

9. The monomer of claim 1, having the general formula: 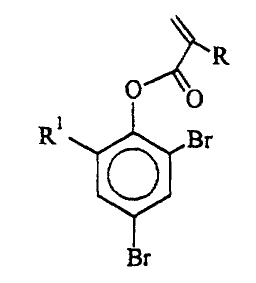 wherein R and R¹ are as defined.

10. The monomer of claim 9, wherein R¹ is a straight or branched alkyl having from 3 to 4 carbons.

11. The monomer of claim 10, wherein the monomer comprises 4,6-dibromo-2-sec-butyl phenolic ester (meth)acrylate.

12. The monomer of claim 10, wherein the monomer comprises 4,6-dibromo-2-tert-butyl phenolic ester (meth)acrylate.

13. The monomer of claim 10, wherein the monomer comprises 4,6-dibromo-2-isopropyl phenolic ester (meth)acrylate.

14. A polymerizable composition comprising the monomer of claim 1.

15. A polymer comprising monomeric units derived from the monomer of claim 1.

16. An optical product prepared from the polymer of claim 15.

## Patentansprüche

1. Monomer der allgemeinen Formel: 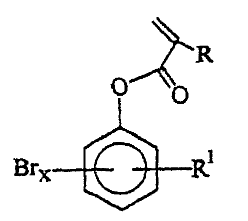 in der x 1 bis 4 ist, R -H oder -CH₃ ist und R¹ ein linearer oder verzweigter Alkylrest mit mindestens zwei Kohlenstoffatomen ist, und wobei das Monomer einen Brechungsindex von mindestens 1,55 aufweist.

2. Monomer nach Anspruch 1, wobei der Brechungsindex mindestens 1,555 beträgt.

3. Monomer nach Anspruch 1, wobei der Brechungsindex mindestes 1,56 beträgt.

4. Monomer nach Anspruch 1, wobei der Schmelzpunkt des Monomers unter 60°C liegt.

5. Monomer nach Anspruch 1, wobei der Schmelzpunkt des Monomers unter 30°C liegt.

6. Monomer nach Anspruch 1, wobei der Schmelzpunkt des Monomers im Bereich von 20°C bis 25°C liegt.

7. Monomer nach Anspruch 1, wobei R¹ ein linearer oder verzweigter Alkylrest mit 3 bis 4 Kohlenstoffatomen ist.

8. Monomer nach Anspruch 7, wobei x 2 ist.

9. Monomer nach Anspruch 1 der allgemeinen Formel: 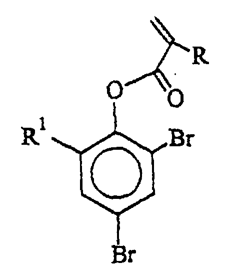 wobei R und R¹ die vorstehend angegebene Bedeutung haben.

10. Monomer nach Anspruch 9, wobei R¹ ein linearer oder verzweigter Alkylrest mit 3 bis 4 Kohlenstoffatomen ist.

11. Monomer nach Anspruch 10, wobei das Monomer (Meth)acrylsäure-4,6-dibrom-2-sek-butylphenolester umfasst.

12. Monomer nach Anspruch 10, wobei das Monomer (Meth)acrylsäure-4,6-dibrom-2-tert-butylphenolester umfasst.

13. Monomer nach Anspruch 10, wobei das Monomer (Meth)acrylsäure-4,6-dibrom-2-isopropylphenolester umfasst.

14. Polymerisierbare Masse, umfassend das Monomer nach Anspruch 1.

15. Polymer, umfassend vom Monomer nach Anspruch 1 abgeleitete Monomereinheiten.

16. Optisches Produkt, hergestellt aus dem Polymer nach Anspruch 15.

## Revendications

1. Monomère de formule générale : 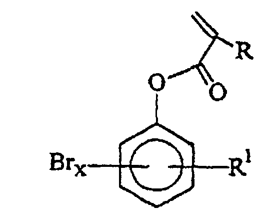 dans laquelle x vaut de 1 à 4, R est -H ou -CH₃, et R¹ est un groupe alkyle à chaîne droite ou ramifiée ayant au moins deux atomes de carbone, et dans lequel le monomère possède un indice de réfraction d'au moins 1,55.

2. Monomère selon la revendication 1, dans lequel l'indice de réfraction est d'au moins 1,555.

3. Monomère selon la revendication 1, dans lequel l'indice de réfraction est d'au moins 1,56.

4. Monomère selon la revendication 1, dans lequel le point de fusion du monomère est inférieur à 60°C.

5. Monomère selon la revendication 1, dans lequel le point de fusion du monomère est inférieur à 30°C.

6. Monomère selon la revendication 1, dans lequel le point de fusion du monomère est dans la gamme de 20°C à 25°C.

7. Monomère selon la revendication 1, dans lequel R¹ est un groupe alkyle à chaîne droite ou ramifiée ayant de 3 à 4 atomes de carbone.

8. Monomère selon la revendication 7, dans lequel x vaut 2.

9. Monomère selon la revendication 1, ayant la formule générale : 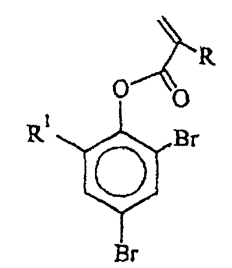 dans lequel R et R¹ sont tels que définis.

10. Monomère selon la revendication 9, dans lequel R¹ est un groupe alkyle à chaîne droite ou ramifiée ayant de 3 à 4 atomes de carbone.

11. Monomère selon la revendication 10, dans lequel le monomère est le (méth)acrylate de 4,6-dibromo-2-sec-butyl-phénol.

12. Monomère selon la revendication 10, dans lequel le monomère est le (méth)acrylate de 4,6-dibromo-2-tert-butyl-phénol.

13. Monomère selon la revendication 10, dans lequel le monomère est le (méth)acrylate de 4,6-dibromo-2-isopropylphénol.

14. Composition polymérisable comprenant le monomère de la revendication 1.

15. Polymère comprenant des motifs monomères dérivés du monomère de la revendication 1.

16. Produit optique préparé à partir du polymère de la revendication 15.
